(19) **Europäisches Patentamt / European Patent Office / Office européen des brevets**

(11) **EP 4 636 072 A1**

(12) **EUROPEAN PATENT APPLICATION**
published in accordance with Art. 153(4) EPC

(43) Date of publication:
**22.10.2025 Bulletin 2025/43**

(21) Application number: **23903393.9**

(22) Date of filing: **06.12.2023**

(51) International Patent Classification (IPC):
**C12N 1/00** $^{(2006.01)}$   **C12M 1/00** $^{(2006.01)}$
**C12N 5/0775** $^{(2010.01)}$   **G06N 20/10** $^{(2019.01)}$

(52) Cooperative Patent Classification (CPC):
**C12M 1/00; C12N 1/00; C12N 5/06; G06N 20/10**

(86) International application number:
**PCT/JP2023/043739**

(87) International publication number:
**WO 2024/128105 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.12.2022 JP 2022199227**

(71) Applicant: **Resonac Corporation
Tokyo 105-7325 (JP)**

(72) Inventors:
- **KAWAI, Tatsuhiko
  Tokyo 105-7325 (JP)**
- **OKUNO, Yoshishige
  Tokyo 105-7325 (JP)**
- **UEDA, Kazuki
  Tokyo 105-7325 (JP)**
- **KOBAYASHI, Momoko
  Tokyo 105-7325 (JP)**
- **NAKAGAWA, Fumiko
  Tokyo 105-7325 (JP)**

(74) Representative: **Strehl & Partner mbB
Maximilianstrasse 54
80538 München (DE)**

(54) **METHOD FOR PRODUCING CELL AGGREGATE, PROPOSING DEVICE, PROPOSING METHOD AND PROGRAM**

(57)    A method of producing cell aggregates includes culturing cells under shear stress of 0.0145 Pa or greater and 0.125 Pa or less.

## FIG.1A

EP 4 636 072 A1

**Description**

TECHNICAL FIELD

The present invention relates to methods of producing cell aggregates, suggestion devices, suggestion methods, and programs.

BACKGROUND ART

Cell aggregates, such as spheroids and the like, have been attracting attention in the field of cell therapy or the like, as cells are aggregated to form the cell aggregates having three-dimensional structures as if they are in vivo.
Regarding production of cell aggregates, for example, Patent Document 1 describes a method of culturing proximal tubule epithelial cells for 5 days to 14 days while repeating stirring and standing using a spinner flask equipped with a stirring blade. Non-Patent Document 1 describes a method of culturing mesenchymal stem cells for 6 days using a bioreactor equipped with an impeller. Non-Patent Document 2 describes a method of shake-culturing mesenchymal stromal/stem cells for one month to 2 months.
In addition, Non-Patent Document 2 describes spheroids in size of approximately 500 $\mu$m to approximately 1,200 $\mu$m.
On the other hand, it is generally known that necrosis is often observed in a central portion of a spheroid due to the limitation in the substance exchange capability, such as insufficient supply of oxygen or nutrients, or insufficient discharged of accumulated waste products.

RELATED ART DOCUMENTS

PATENT DOCUMENTS

Patent Document 1: Japanese Laid-Open Patent Publication No. 2021-191305

NON-PATENT DOCUMENTS

Non-Patent Document 1: Bioengineering 2017, 4, 47
Non-Patent Document 2: Frontiers in Bioengineering and Biotechnology 2020, 8,590332

DISCLOSURE OF THE INVENTION

PROBLEMS TO BE SOLVED BY THE INVENTION

The technologies of the related art are complicated methods, such as using a spinner flask equipped with a stirring blade, or a bioreactor equipped with an impeller, or culturing while repeating stirring and resting phases, and are not simple and efficient methods as culturing needs to be performed for a long period. Therefore, the methods of the related art are not simple and efficient methods. In addition, a center portion of a large-size spheroid does not provide a desirable culturing environment for cells.
One aspect of the present disclosure aims to provide a method of simply and efficiently producing cell aggregates.

MEANS FOR SOLVING THE PROBLEMS

The present disclosure includes the following configurations.

[1] A method of producing cell aggregates includes culturing cells under shear stress of 0.0145 Pa or greater and 0.125 Pa or less.
[2] The method of producing the cell aggregates described in [1] above, in which the cell aggregates obtained by the culturing have a number mean diameter of 400 $\mu$m or less.
[3] A suggestion device includes a condition input part, a condition-value determination part, and a condition output part. The condition input part is configured to receive input of preconditions in which one or more condition values among culturing conditions for producing cell aggregates are undetermined. The condition-value determination part is configured to determine the one or more condition values so that an indicator calculated based on the preconditions satisfies a target value that is determined in advance. The condition output part is configured to output suggested conditions including the one or more condition values that are determined.

[4] The suggestion device described in [3] above, in which the culturing conditions are conditions for shaking a vessel accommodating both cells and a medium to produce the cell aggregates, and the one or more condition values include a kind of a vessel, a kind of the medium, an amount of the medium, or a rotational speed for shaking the vessel.

[5] The suggestion device described in [4] above, in which the indicator is shear stress applied to the cells.

[6] The suggestion device described in [5] above, in which the target value is 0.0145 Pa or greater and 0.125 Pa or less.

[7] The suggestion device described in any one of [3] to [6] above, in which the condition-value determination part is configured to determine the one or more condition values using a learned model that has learned a relationship between the culturing conditions and the indicator.

[8] The method of producing the cell aggregates described in [1] or [2] above further includes shaking a vessel in which both the cells and a medium are accommodated according to the suggested conditions output by the suggestion device described in any one of [3] to [7] above.

[9] A suggestion method includes causing a computer to perform: a process of receiving input of preconditions in which one or more condition values are undetermined among culturing conditions for producing cell aggregates; a process of determining the one or more condition values so that an indicator calculated based on the preconditions satisfies a target value that is determined in advance; and a process of outputting suggested conditions including the one or more condition values determined.

[10] A program for causing a computer to perform: a process of receiving input of preconditions in which one or more condition values are undetermined among culturing conditions for producing cell aggregates; a process of determining the one or more condition values so that an indicator calculated based on the preconditions satisfies a target value that is determined in advance; and a process of outputting suggested conditions including the one or more condition values determined.

EFFECTS OF THE INVENTION

According to one aspect of the present disclosure, a method of simply and efficiently producing cell aggregates can be provided.

BRIEF DESCRIPTION OF THE DRAWINGS

[Fig. 1A] Fig. 1A is a photograph capturing cell aggregates obtained in Experiment 1.
[Fig. 1B] Fig. 1B is a photograph capturing cell aggregates obtained in Experiment 2.
[Fig. 1C] Fig. 1C is a photograph capturing cell aggregates obtained in Experiment 3.
[Fig. 1D] Fig. 1D is a photograph capturing cell aggregates obtained in Experiment 4.
[Fig. 2A] Fig. 2A is a photograph capturing cell aggregates obtained in Example 1.
[Fig. 2B] Fig. 2B is a photograph capturing cell aggregates obtained in Example 2.
[Fig. 2C] Fig. 2C is a photograph capturing cell aggregates obtained in Example 3.
[Fig. 2D] Fig. 2D is a photograph capturing cell aggregates obtained in Example 4.
[Fig. 2E] Fig. 2E is a photograph capturing cell aggregates obtained in Example 5.
[Fig. 2F] Fig. 2F is a photograph capturing cell aggregates obtained in Comparative Example 1.
[Fig. 2G] Fig. 2G is a photograph capturing cell aggregates obtained in Comparative Example 2.
[Fig. 2H] Fig. 2H is a photograph capturing cell aggregates obtained in Comparative Example 3.
[Fig. 2I] Fig. 2I is a photograph capturing cell aggregates obtained in Comparative Example 4.
[Fig. 3] Fig. 3 is a block diagram illustrating one example of an overall configuration of a culturing-condition suggestion system.
[Fig. 4] Fig. 4 is a block diagram illustrating one example of a hardware configuration of a computer.
[Fig. 5] Fig. 5 is a block diagram illustrating one example of a functional configuration of the culturing-condition suggestion system of Embodiment 1.
[Fig. 6] Fig. 6 is a flowchart illustrating one example of a process flow of the suggestion method of Embodiment 1.
[Fig. 7] Fig. 7 is a diagram illustrating one example of setting information.
[Fig. 8] Fig. 8 is a diagram illustrating one example of target information.
[Fig. 9] Fig. 9 is a block diagram illustrating one example of a functional configuration of the culturing-condition suggestion system of Embodiment 2.
[Fig. 10] Fig. 10 is a flowchart illustrating one example of a process flow of the suggestion method of Embodiment 2.

MODE OF CARRYING OUT THE INVENTION

(Method of producing cell aggregates)

The method of producing cell aggregates includes a culturing step, and may further include other steps.

-Culturing step-

The culturing step is a step of culturing cells under shear stress of 0.0145 Pa or greater and 0.125 Pa or less.

When the shear stress is within the above range, cell aggregates which reduced the limitation in substance exchange capability of a center portion of each of the cell aggregates, such as insufficient supply of oxygen or nutrients, or insufficient discharge of accumulated waste products, can be produced.

A lower limit of the shear stress is not particularly limited as long as the lower limit is 0.0145 Pa or greater, and may be appropriately selected according to the intended purpose. From the viewpoint of more efficient production of desired cell aggregates, the lower limit of the shear stress is preferably 0.0150 Pa or greater, 0.0180 Pa or greater, 0.0200 Pa or greater, 0.0220 Pa or greater, 0.0250 Pa or greater, or 0.0270 Pa or greater.

An upper limit of the shear stress is not particularly limited as long as the upper limit is 0.125 Pa or less, and may be appropriately selected according to the intended purpose. From the viewpoint of more efficient production of desired cell aggregates, the upper limit of the shear stress is preferably 0.120 Pa or less, 0.115 Pa or less, 0.110 Pa or less, 0.105 Pa or less, 0.100 Pa or less, or 0.095 Pa or less.

Among the above ranges, the shear stress is preferably 0.0150 Pa or greater and 0.120 Pa or less, 0.0180 Pa or greater and 0.115 Pa or less, 0.0200 Pa or greater and 0.110 Pa or less, 0.0220 Pa or greater and 0.105 Pa or less, 0.0250 Pa or greater and 0.100 Pa or less, or 0.0270 Pa or greater and 0.095 Pa or less, from the viewpoint of efficient production of cell aggregate.

The shear stress $\tau$ (Pa) is defined by the following equation (1).
[Math. 1]

$$\tau = \mu \cdot \dot{\gamma} \quad \cdots (1)$$

The shear stress is determined using computational fluid dynamic (CFD) simulation (Fluent (registered trademark) of ANSIS). The shear stress is calculated based on a shape and size of a vessel, an amount and viscosity of a medium, and a rotational speed for shaking the vessel. Specifically, a mesh simulating a shape and size of a vessel and an amount of a medium is created, and as parameters, a viscosity of the medium, a shaking rotational speed, and liquid-surface boundary conditions are set. Then, calculation is performed according to the volume of fluid (VOF) method, and an average of the shear stress values of the mesh cells obtained by convergence calculation is determined, thereby calculating shear stress.

A method of culturing cells under the shear stress is not particularly limited, and may be appropriately selected according to the intended purpose. Examples of the method include: a method in which a culture vessel is set on a shaker, and culturing is performed by shaking the culture vessel itself; a method in which culturing is performed while stirring with a stirring blade or a stirring bar placed in a culture vessel; a method in which culturing is performed in a culture vessel equipped with a stirring blade or a stirring bar, such as a spinner flask or the like; a method in which culturing is performed using a bioreactor; and the like. Among above methods, the method in which the culture vessel is set on the shaker and culturing is performed by shaking the culture vessel itself is preferred from the viewpoint of increasing the chance of cell contact and enabling the simple and efficient production of desired cell aggregates. Examples of a system of shaking include reciprocating, rotating, and the like. From the viewpoint of increased chances of contact between cells, the movement of rotating (rotary shaking system) can be selected.

A driving diameter of the rotary shaking system is not particularly limited, and may be appropriately selected according to the intended purpose. Examples of the driving diameter include 10 mm or greater and 30 mm or less.

In the method in which the culture vessel is set on the shaker and shake culture is performed, the shear stress can be adjusted to the above range by controlling a shape and capacity of the culture vessel, an amount of a medium added to the culture vessel, and a rotational speed for shaking the shaker.

In the method in which culturing is performed while stirring with a stirring blade or a stirring bar placed in a culture vessel, the shear stress can be adjusted to the above range by controlling a shape and capacity of the culture vessel, an amount of a medium added to the culture vessel, and a rotational speed of the stirring blade or the stirring bar.

In the case where shake culture is performed in a rotary shaking system having a driving diameter of 19 mm, the following conditions can be selected when a culture vessel of each of the following capacities is placed on the shaker to culture.

·When a 125 mL flask is used as the culture vessel and 12.5 mL of a medium is added, the rotational speed for shaking the shaker can be set at 40 rpm to 65 rpm.

·When a 125 mL flask is used as the culture vessel and 30 mL of a medium is added to the culture vessel, the rotational speed for shaking the shaker can be set at 65 rpm to 110 rpm.

·When a 250 mL flask is used as the culture vessel and 20 mL of a medium is added to the culture vessel, the rotational speed for shaking the shaker can be set at 40 rpm to 70 rpm.

·When a 250 mL flask is used as the culture vessel and 30 mL of a medium added to the culture vessel, the rotational speed for shaking the shaker can be set at 40 rpm to 100 rpm.

·When a 250 mL flask is used as the culture vessel and 50 mL of a medium is added to the culture vessel, the rotational speed for shaking the shaker can be set at 10 rpm to 75 rpm.

·When a 2 L flask is used as the culture vessel and 200 mL of a medium is added to the culture vessel, the rotational speed for shaking the shaker can be set at 40 rpm to 60 rpm.

·When a 2 L flask is used as the culture vessel and 400 mL of a medium is added to the culture vessel, the rotational speed for shaking the shaker can be set at 45 rpm to 65 rpm.

The shaker is not particularly limited, and may be appropriately selected according to the intended purpose. Examples of the shaker include orbital shakers and the like.

In the culturing step, duration for application of shearing is not particularly limited as long as cells can be cultured under the above shear stress. The duration may be appropriately selected according to the intended purpose. From the viewpoint of simple and efficient production of desired cell aggregates, the shear stress can be continuously applied in the culturing step. From the viewpoint of adjustment of a size of cell aggregates, the application of the shearing can be optionally stopped for a short period.

Duration for stopping the application of the shearing is not particularly limited, and may be appropriately selected according to the intended purpose. From the viewpoint of simple and efficient production of desired cell aggregates, the duration can be 5 minutes or less, or 3 minutes or less.

The vessel is not particularly limited, and may be selected from vessels typically used for production of cell aggregates. Examples of the vessel include flasks, bottles, plates, and the like.

The flasks are not particularly limited, and may be appropriately selected according to the intended purpose. Examples of the flasks include Erlenmeyer flasks, round-bottom flasks, flat-bottom flasks, and the like.

The cells are not particularly limited as long as the cell are cells that can form cell aggregate. The cells may be appropriately selected according to the intended purpose. From the viewpoint of efficient production of cell aggregates, examples of the cells include eukaryotic cells, such as plant cells and animal cells, and the like. The animal cells may be mammalian cells, and examples of the mammalian cells includes cells of humans, monkeys, chimpanzees, cows, pigs, horses, sheep, goats, rabbits, rats, mice, guinea pigs, dogs, cats, and the like.

A kind of the animal cells are not particularly limited, as long as the animal cells are cells that come into contact with each other in a growth phase or any other periods to form cell aggregates, and may be appropriately selected according to the intended purpose. Examples of the kind of the animal cells include embryonic stem cells (ES cells), embryonic carcinoma cells (EC cells), embryonic germ stem cells (EG cells), nuclear-transfer ES cells, somatic cellderived ES cells (ntES cells), artificial stem cells, induced pluripotent stem cells (iPS cells), hematopoietic stem cells, mesenchymal stem cells, and the like.

The mesenchymal stem cells are not particularly limited, and may be appropriately selected according to the intended purpose. Examples of the mesenchymal stem cells include adipose-derived mesenchymal stem cells, bone marrow-derived mesenchymal stem cells, placenta-derived mesenchymal stem cells, umbilical cord-derived mesenchymal stem cells, dental pulp-derived mesenchymal stem cells, and the like.

The number of the cells is not particularly limited, and may be appropriately selected according to the intended purpose. From the viewpoint of efficient production of cell aggregates, the number of cells before culturing (seeding cell number) is preferably $0.01 \times 10^6$ cells/mL or greater and $5 \times 10^6$ cells/mL or less, $0.05 \times 10^6$ cells/mL or greater and $1 \times 10^6$ cells /mL or less, or $0.05 \times 10^6$ cells/mL or greater and $0.8 \times 10^6$ cells/mL or less. As a seeding density is reduced, cell aggregates having a small value of coefficient of variation (CV) are likely to be obtained, and therefore the seeding density can be appropriately selected according to the intended purpose.

The culture medium for the cells is not particularly limited as long as the culture medium is a culture medium that can be typically used for culturing selected cells. The culture medium may be appropriately selected according to the intended purpose. The culture medium can include any additive components. Examples of the additive components include additive components typically added to a medium, such as inorganic salts, sugars, amino acids, protein, serum, serum substitutes, vitamins, hormones, antibiotics, growth factors, biological tissue extract, and the like. The medium may or may not include serum. In the case where the medium does not include serum, the medium may include a serum substitute. The medium may be a medium including various raw materials or a complete synthetic medium that does not include various raw materials. As the medium, any known basal medium as a basal medium for culturing cells, or any other known media can be used.

The basal medium of the culture medium is not particularly limited, and may be appropriately selected according to the intended purpose. Example of the basal medium include an MEM (Eagle's minimal essential medium), an αMEM medium (α-modified Eagle's minimal essential medium), a StemPro (registered trademark) MSC SFM medium (Thermo Fisher Scientific Inc., A1050901), a PRIME-XV MSC XSFM MDF1 medium, and the like.

A viscosity of the culture medium for the cells is not particularly limited as long as the viscosity is within a range typically used for culturing of selected cells. The viscosity may be appropriately selected according to the intended purpose. From the viewpoint of efficient production of desired cell aggregates, the viscosity can be 0.0001 kg/(m·s) or greater and 0.01 kg/(m·s) or less, and can be 0.0001 kg/(m·s) or greater and 0.005 kg/(m·s) or less. As a measurement method of the viscosity, electromagnetic spinning (EMS) is used, and the viscosity is a viscosity measured at a constant temperature of 37°C and at a rotational speed of a motor being 500 rpm, 600 rpm, 700 rpm, 800 rpm, 900 rpm, or 1,000 rpm.

A lower limit of the number of days of the culturing is not particularly limited, and may be appropriately selected according to a state of the cells. From the viewpoint of efficient production of desired cell aggregates, the lower limit of the number of days of the culturing can be 1 or more days, or 2 or more days.

An upper limit of the number of days of the culturing is not particularly limited, and may be appropriately selected according to the intended purpose. From the viewpoint of simple and efficient production of cell aggregates, the upper limit of the days of the culturing can be 10 or less days, 8 or less days, or 5 or less days.

Among the above ranges, the number of days of the culturing can be 1 or more days and 10 or less days, 1 or more days and 8 or less days, 1 or more days and 5 or less days, or 2 or more days and 5 or less days, from the viewpoint of simple and efficient production of cell aggregates.

A method of the culturing is not particularly limited, and may be appropriately selected according to the intended purpose. From the viewpoint of simple and efficient production of desired cell aggregates, the method is preferably suspension culture, or preferably culturing that does not use a support (scaffold).

The cell aggregates are not particularly limited, and may be appropriately selected according to the intended purpose. Examples of the cell aggregates include spheroids, organoids, and the like.

An upper limit of the number mean diameter is not particularly limited, and may be appropriately selected according to the intended purpose. From the viewpoint of an internal environment in each of cell aggregates, the upper limit of the number mean diameter can be 400 μm or less, 350 μm or less, 300 μm or less, 250 μm or less, 200 μm or less, 180 μm or less, 150 μm or less, 130 μm or less, 110 μm or less, or 100 μm or less.

A lower limit of the number mean diameter is not particularly limited, and may be appropriately selected according to the intended purpose. From the viewpoint of handling as cell aggregates, the lower limit of the number mean diameter can be 10 μm or greater, or 20 μm or greater.

The number mean diameter is measured by the following method.

The number mean diameter of the cell aggregates can be determined by measuring diameters (particle diameters) of cell aggregates, which are measurement targets, observed under an optical microscope. In the present disclosure, an equivalent circle diameter of the cell aggregate of the measurement target, i.e., $(4 \times \text{area}/\pi)^{0.5}$, is measured from the image obtained by the optical microscopic observation using image analysis software "ImageJ" (provided by National Institute of Health, USA, which will be omitted hereinafter), and the measured value is determined as a size of the cell aggregate of the measurement target. As the number of the cell aggregates to be measured, 100 cell aggregates are taken from the minimum diameter up to 95% in the cumulative distribution based on the diameter, and a mean value of the 100 cell aggregates is determined.

From the viewpoint of homogeneity, handling, and the like of the cell aggregates, a value of CV (standard deviation/mean value) of diameters of the cell aggregates determined from the standard deviation and the mean value of the number mean diameter of the cell aggregates can be 0.80 or less, 0.60 or less, 0.40 or less, or 0.35 or less.

Circularity of the cell aggregates is not particularly limited, and may be appropriately selected according to the intended purpose. From the viewpoint of production of cell aggregates having center portions suitable as a culturing environment for cells, the circularity is preferably 5 or less, 3 or less, or 2 or less.

The circularity is measured by the following method.

The circularity of the cell aggregates can be determined by measuring diameters (particle diameters) of the cell aggregates, which are measurement targets, observed under an optical microscope. In the present disclosure, a value of (length of outline of measured cell aggregate)$^2$/(4 × π × area) is measured from the image obtained by the optical microscopic observation using image analysis software "ImageJ" and the measured value is determined as the circularity of the cell aggregate of the measurement target. As the number of the cell aggregates to be measured, 100 cell aggregates are taken from the minimum diameter up to 95% in the cumulative distribution based on the diameter, and a mean value of the 100 cell aggregates is determined.

-Other steps-

The above other steps are not particularly limited, and may be appropriately selected according to the intended purpose. Examples of the other steps include a cell collecting step after the culturing step.

--Cell collecting step--

The cell collecting step is not particularly limited, and may be appropriately selected according to the intended purpose. Examples of the cell collecting step include: a method in which a culture solution obtained in the culturing step is subjected to centrifugation to remove a supernatant; a method in which cells are separated by filtration using an appropriate filter; and the like.

(Culturing method)

The culturing method is a method including culturing of cells under shear stress of 0.0145 Pa or greater and 0.125 Pa or less.
The culturing method includes a culturing step, and may further include other steps.
The culturing step and other steps are as described in the above (Method of producing cell aggregates).

Examples

Examples of the present invention will be described hereinafter, but the present invention shall not be construed as being limited to these examples.

<Production Example 1: Culture expansion of mesenchymal stem cells and production of cell line stock>

Adipose-derived mesenchymal stem cells (Adipose-Derived Stem Cells; ADSC, PT-5006) acquired from Lonza were thawed. The thawed cells were washed with D-PBS(-) (FUJIFILM Wako Pure Chemical Corporation, 045-29795), followed by centrifugation (Eppendorf Himac Technologies Co., Ltd., CR22N) for 3 minutes at $300 \times g$. The resultant cell pellet was suspended in a PRIME-XV MSC XSFM MDF1 medium (552-37463, FUJIFILM Irvine Scientific, Inc.), and the number of the cells in the medium was measured by a cell counter NC-200 (ChemoMetec Inc.) according to the product protocol using Via1-Cassette (941-0012, ChemoMetec Inc.). The cells were seeded in a T175 flask (VTC-F175V, VIOLAMO) at 3,000 cells/cm$^2$ to expand the culture. The second passage cells were suspended in STEM-CELLBANKER (registered trademark) (CB045, Takara Bio Inc.), and the suspension was dispensed into each of cryotubes by $1 \times 10^6$ cells/1 mL. The cryotubes were placed in a bio freezing vessel, BICELL, and were cryopreserved at -80°C. The cryopreserved cells were transferred to a liquid nitrogen tank on the following day or later to store in a vapor phase at -195°C or lower.

<Production Example 2: Production of cell suspension>

The cell line stock produced in Production Example 1 was expanded in a T175 flask (VTC-F175V, VIOLAMO) using 25 mL of a PRIME-XV MSC XSFM MDF1 medium (552-37463, FUJIFILM Irvine Scientific, Inc.). The fifth passage cells were used for an agitation culture test in Experiments 1 to 4, Examples, and Comparative Examples below.
The fifth passage cells adhered to the T175 flask (VTC-F175V, VIOLAMO) were washed with 10 mL of D-PBS(-) (045-29795, FUJIFILM Wako Pure Chemical Corporation), and 5 mL of a cell dissociation reagent, Accutase (AT-104, Innovative Cell Technologies, Inc.) was added, followed by incubating for 5 minutes at 37°C. The dissociated cells were suspended in 25 mL of D-PBS(-) (045-29795, FUJIFILM Wako Pure Chemical Corporation) and collected in a 50 mL centrifuge tube (50050, SPL LIFE SCIENCES). Centrifugation (CR22N, Eppendorf Himac Technologies Co., Ltd.) was performed for 3 minutes at $300 \times g$, the supernatant was removed, and the resultant pellet was tapped and again suspended in 10 mL of D-PBS(-) (045-29795, FUJIFILM Wako Pure Chemical Corporation). The number of cells in the resultant suspension was counted by a cell counter NC-200 (ChemoMetec Inc.) according to the product protocol using Vial-Cassette (941-0012, ChemoMetec Inc.).

1. Screening of optimal culturing condition

<Experiment 1>

The cell suspension (12.5 mL) having the cell number of $1 \times 10^7$, which was produced in Production Example 2, was added to a 15 mL centrifuge tube, Falcon (registered trademark) conical tube (352096, Corning Japan, K.K.), and centrifugation (Eppendorf Himac Technologies Co., Ltd., CR22N) was performed for 3 minutes at 300×g, followed by removing the supernatant. The resultant cell pellet was tapped and suspended in 12.5 mL of a STEMPRO (registered trademark) MSC SFM medium (Thermo Fisher Scientific Inc., A1050901). The resultant cell suspension was seeded in a 125 mL Erlenmeyer flask (781211, NEST Biotechnology Co., Ltd.). Then, shake culture was performed for 3 days at the rotational speed of 50 rpm on a shake agitator, LabServ digital orbital shaker (FS120460LJ, Thermo Fisher Scientific Inc.) placed in a $CO_2$ incubator (MCO-170AICUVD, PHC Corporation) set at 37°C.

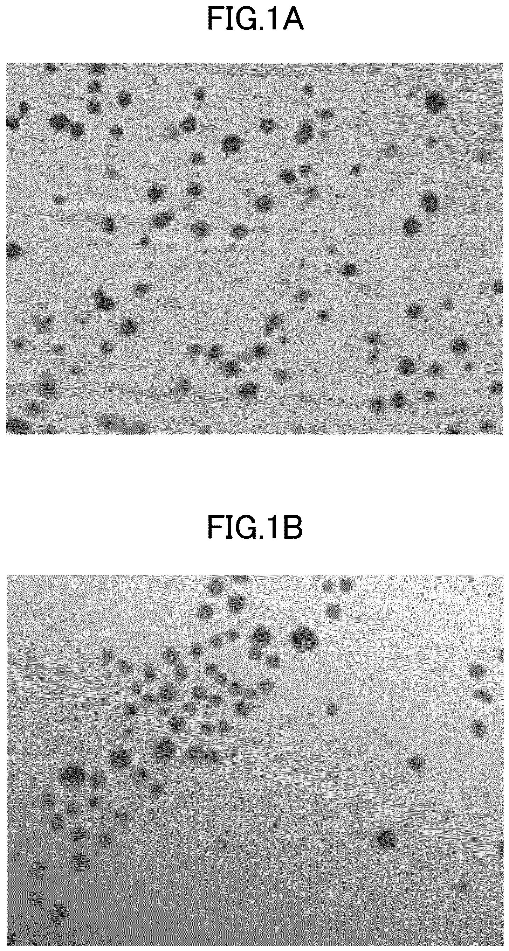

After the shake culture, the resultant culture was observed at the magnification of 40× using a phase-contrast microscope (Olympus CKX53) from which a condenser was removed, and a photograph was taken. The result is presented in Fig. 1A.

In addition, the number mean diameter and circularity of the obtained cell aggregates were measured in the following manner, and the shear stress was calculated. As presented in Table 1, the cell aggregates having the number mean diameter of 39 μm and the circularity of 1.78 were produced. The value of CV of the number mean diameter was 0.31. The shear stress applied during the production of the cell aggregates was 0.043 Pa.

-Number mean diameter

The number mean diameter of the cell aggregates were determined by measuring diameters (particle diameters) of cell aggregates, which were measurement targets, observed under an optical microscope. An equivalent circle diameter of the cell aggregate of the measurement target, i.e., $(4 \times \text{area} / \pi)^{0.5}$, observed under the optical microscope was determined as size of the cell aggregate of the measurement target. As the number of the cell aggregates to be measured, 100 cell aggregates were taken from the minimum diameter up to 95% in the cumulative distribution based on the diameter, and a mean value of the 100 cell aggregates was determined. Image analysis software "ImageJ" was used for the measurement of the number mean diameter.

-Measurement of circularity-

The circularity of the cell aggregates were determined by measuring diameters (particle diameters) of the cell aggregates, which were measurement targets, observed under an optical microscope. A value of (length of outline of measured cell aggregate)$^2$/(4 × π × area) observed under the optical microscope was determined as the circularity of the cell aggregate of the measurement target. As the number of the cell aggregates to be measured, 100 cell aggregates were taken from the minimum diameter up to 95% in the cumulative distribution based on the diameter, and a mean value of the 100 cell aggregates was determined. Image analysis software "ImageJ" was used for the measurement of the circularity.

-Calculation of shear stress-

The shear stress was determined using computational fluid dynamic (CFD) simulation (Fluent (registered trademark) of ANSIS). The shear stress was calculated based on the shape and size of the vessel, the amount and viscosity of the medium, and the rotational speed for shaking the vessel. Specifically, the mesh simulating the shape and size of the vessel and the amount of the medium was created, and as parameters, the viscosity of the medium, the shaking rotational speed, and liquid-surface boundary conditions are set. Then, calculation was performed according to the volume of fluid (VOF) method, and an average of the shear stress values of the mesh cells obtained by convergence calculation was determined, thereby calculating shear stress.

<Experiment 2>

Culturing, phase-contrast microscopic observation, measurement of a number mean diameter and circularity of cell aggregates, and calculation of shear stress were performed in the same manner as in Experiment 1, except that the cell pellet having the cell number of $1 \times 10^7$ was suspended in 30 mL of a STEMPRO (registered trademark) MSC SFM medium (A1050901, Thermo Fisher Scientific Inc.), and shake culture was performed at a rotational speed of 70 rpm. The results are presented in Fig. 1B and Table 1. As presented in Table 1, cell aggregates having the number mean diameter of 55 μm and the circularity of 1.76 were produced. The value of CV of the number mean diameter was 0.38. The shear stress during the production of the cell aggregates was 0.029 Pa.

<Experiment 3>

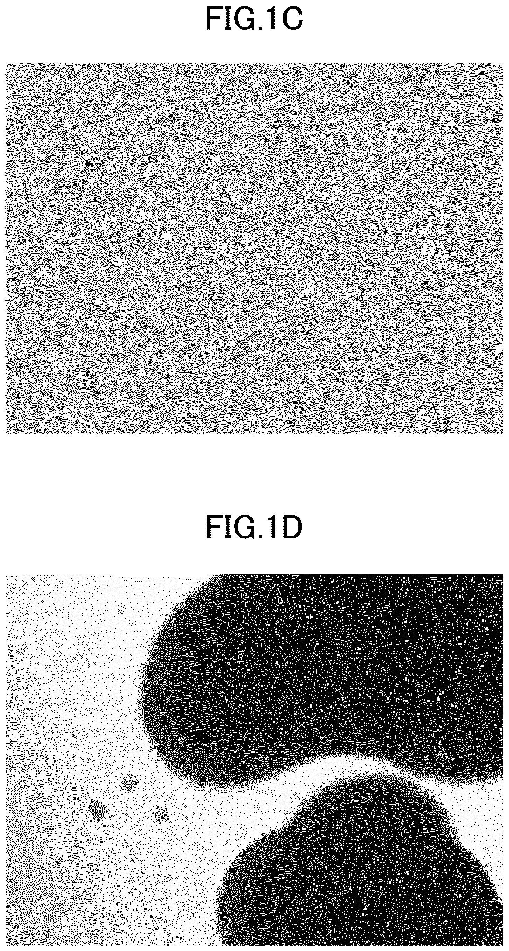

Culturing, phase-contrast microscopic observation, measurement of a number mean diameter and circularity of cell aggregates, and calculation of shear stress were performed in the same manner as in Experiment 1, except that shake culture was performed at a rotational speed of 70 rpm. The results are presented in Fig. 1C and Table 1. As presented in Fig. 1C and Table 1, cell death occurred, and therefore a sufficient number of cell aggregates, which would allow measurement of a number mean diameter and circularity, could not be produced. The shear stress during the production of the cell aggregates was 0.127 Pa. In Table 1, "-" means not measured.

<Experiment 4>

Culturing, phase-contrast microscopic observation, measurement of a number mean diameter and circularity of cell aggregates, and calculation of shear stress were performed in the same manner as in Experiment 1, except that the cell pellet having the cell number of $1 \times 10^7$ was suspended in 30 mL of a STEMPRO (registered trademark) MSC SFM medium (A1050901, Thermo Fisher Scientific Inc.), and the shake culture was performed at a rotational speed of 60 rpm. The results are presented in Fig. 1D and Table 1. As presented in Fig. 1D and Table 1, the number mean diameter of the obtained aggregates was 1,000 $\mu$m or greater, and therefore the obtained aggregates could not be expected to serve as functional cell aggregates. The shear stress during the production of the cell aggregates was 0.014 Pa. In Table 1, "-" means not measured.

[Table 1]

|  | Experiment 1 | Experiment 2 | Experiment 3 | Experiment 4 |
|---|---|---|---|---|
| Vessel | 125 mL Flask | | | |
| Medium amount (mL) | 12.5 | 30 | 12.5 | 30 |
| Shaking rotational speed (rpm) | 50 | 70 | 70 | 60 |
| Seeding cell number ($\times 10^7$) | 1 | 1 | 1 | 1 |
| Number mean diameter ($\mu$m) | 39 | 55 | - | >1000 |
| Value of CV | 0.31 | 0.38 | - | - |
| Circularity | 1.78 | 1.76 | - | - |
| Shear stress (Pa) | 0.043 | 0.029 | 0.127 | 0.014 |

It was found from the results of Table 1 that the size of the cell aggregates changed even with the same amount of the medium, and the size of the obtained cell aggregates changed even with the same shaking rotational speed. Accordingly, it was suggested that the optimal culturing condition could be set by the shear stress, not the amount of the medium nor the rotational speed of shaking.

Next, whether the above range of the shear stress was suitable as a parameter of the optimal culturing condition was verified.

2. Verification Experiment

<Example 1>

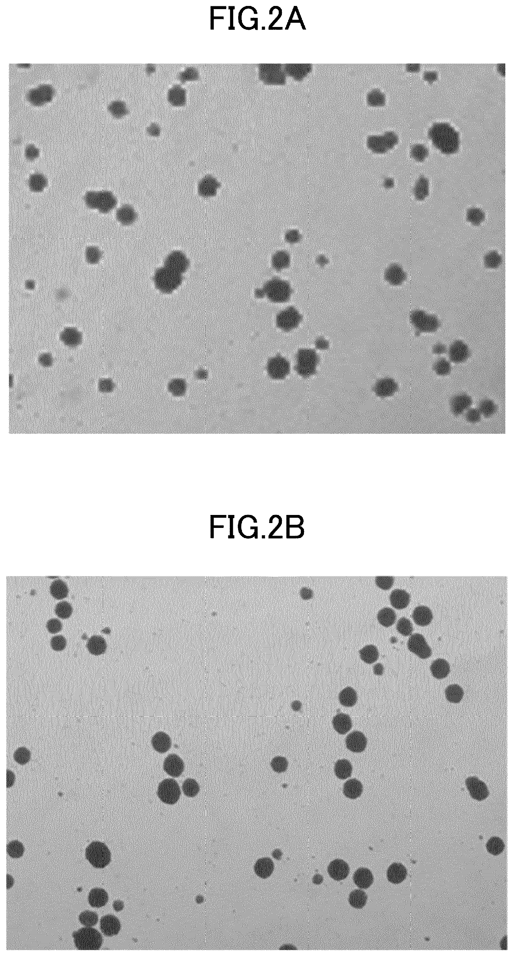

Culturing, phase-contrast microscopic observation, measurement of a number mean diameter and circularity of cell aggregates, and calculation of shear stress were performed in the same manner as in Experiment 1, except that a cell pellet having a cell number of $1 \times 10^7$ was suspended in 20 mL of a STEMPRO (registered trademark) MSC SFM medium (A1050901, Thermo Fisher Scientific Inc.), and the resultant cell suspension was seeded in a 250 mL Erlenmeyer flask (782111, NEST Biotechnology Co., Ltd.). The results are presented in Fig. 2A and Table 2. As presented in Table 2, the cell aggregates having the number mean diameter of 39 $\mu$m and circularity of 1.57 were produced. A value of CV of the number mean diameter was 0.26. The shear stress during the production of the cell aggregates was 0.066 Pa.

<Example 2>

Culturing, phase-contrast microscopic observation, measurement of a number mean diameter and circularity of cell aggregates, and calculation of shear stress were performed in the same manner as in Experiment 1, except that a cell pellet having a cell number of $1 \times 10^7$ was suspended in 30 mL of a STEMPRO (registered trademark) MSC SFM medium (A1050901, Thermo Fisher Scientific Inc.), and the resultant cell suspension was seeded in a 250 mL Erlenmeyer flask (782111, NEST Biotechnology Co., Ltd.). The results are presented in Fig. 2B and Table 2. As presented in Table 2, the cell aggregates having the number mean diameter of 63 $\mu$m and circularity of 1.54 were produced. A value of CV of the number mean diameter was 0.38. The shear stress during the production of the cell aggregates was 0.029 Pa.

<Example 3>

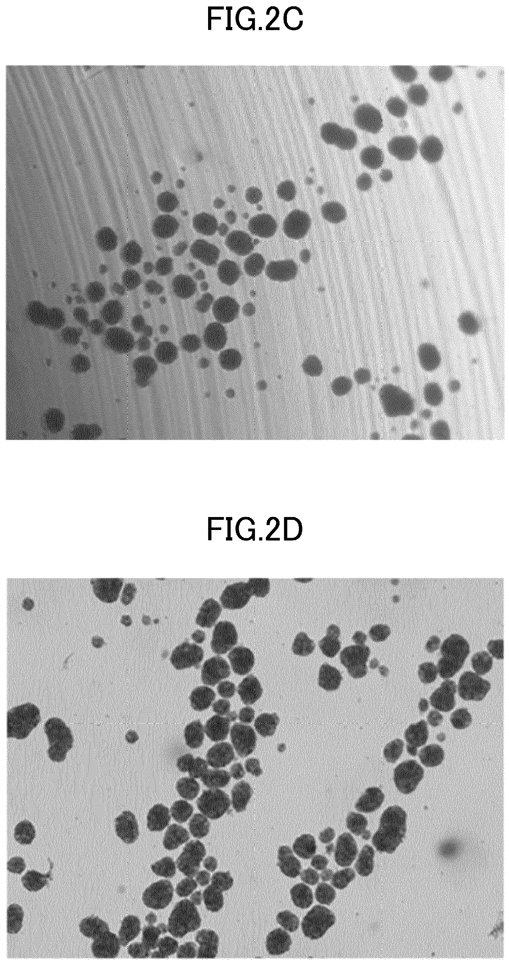

Culturing, phase-contrast microscopic observation, measurement of a number mean diameter and circularity of cell aggregates, and calculation of shear stress were performed in the same manner as in Experiment 1, except that a cell pellet having a cell number of $1.6 \times 10^7$ was suspended in 50 mL of a STEMPRO (registered trademark) MSC SFM medium (A1050901, Thermo Fisher Scientific Inc.), the resultant cell suspension was seeded in a 250 mL Erlenmeyer flask (782111, NEST Biotechnology Co., Ltd.), and shake culture was performed at a rotational speed of 70 rpm. The results are presented in Fig. 2C and Table 2. As presented in Table 2, the cell aggregate having the number mean diameter of 56 $\mu$m and circularity of 1.64 were produced. A value of CV of the number mean diameter was 0.61. The shear stress during the production of the cell aggregates was 0.105 Pa. Fig. 2C illustrates another example of a phase-contrast microscopic photograph where shake culture was performed in a flask having the same capacity with the same amount of a medium.

<Example 4>

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 200 mL of a STEMPRO (registered trademark) MSC SFM medium (A1050901, Thermo Fisher Scientific Inc.), and the resultant cell suspension was seeded in a 2 L Erlenmeyer flask (785011, NEST Biotechnology Co., Ltd.). The results are presented in Fig. 2D and Table 2. As presented in Table 2, the cell aggregates having the number mean diameter of 70 $\mu$m and circularity of 1.71 were produced. A value of CV of the number mean diameter was 0.40. The shear stress during the production of the cell aggregates was 0.093 Pa.

<Example 5>

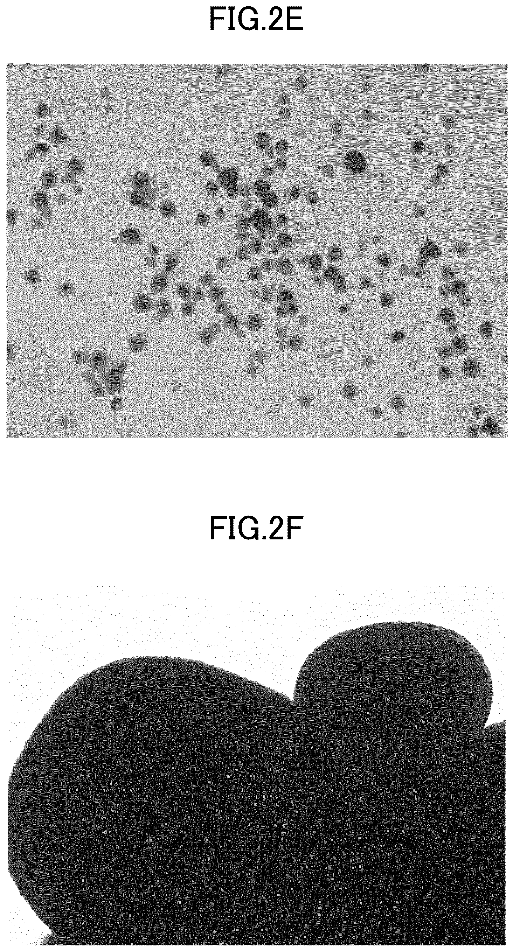

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 400 mL of a STEMPRO (registered trademark) MSC SFM medium (A1050901, Thermo Fisher Scientific Inc.), and the resultant cell suspension was seeded in a 2 L Erlenmeyer flask (785011, NEST Biotechnology Co., Ltd.). The results are presented in Fig. 2E and Table 2. As presented in Table 2, the cell aggregates having the number mean diameter of 49 $\mu$m and circularity of 1.79 were produced. A value of CV of the number mean diameter was 0.29. The shear stress during the production of the cell aggregates was 0.031 Pa.

<Comparative Example 1>

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 200 mL of a STEMPRO (registered trademark) MSC SFM medium (A1050901, Thermo Fisher Scientific Inc.), the resultant cell suspension was seeded in a 2 L Erlenmeyer flask (785011, NEST Biotechnology Co., Ltd.), and shake culture was performed at a rotational speed of 30 rpm. The results are presented in Fig. 2F and Table 2. As presented in Fig. 2F and Table 2, the number mean diameter of the obtained aggregates was 1000 $\mu$m or greater, and therefore it was clear that the obtained aggregates could not be expected to serve as functional cell aggregates. The shear stress during the production of the cell aggregates was 0.0054 Pa. In Table 2, "-" means not measured.

<Comparative Example 2>

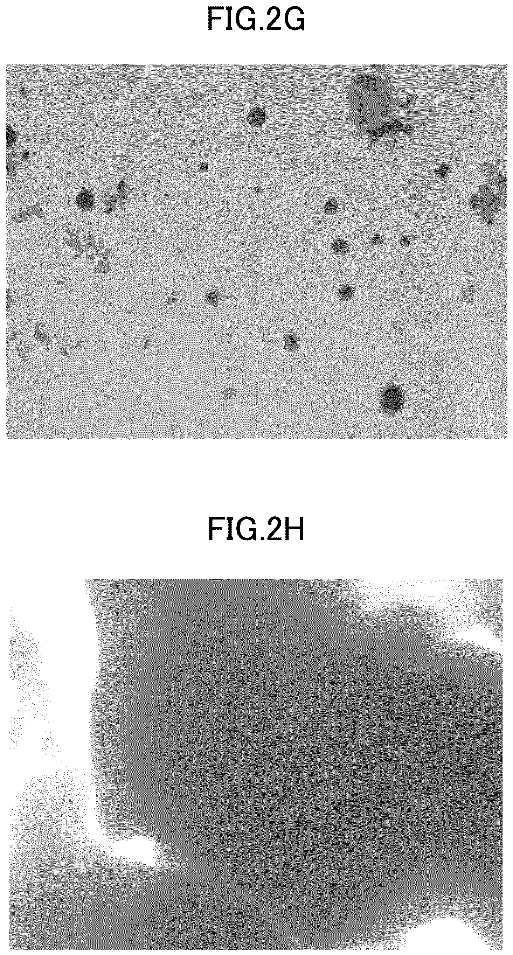

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 200 mL of a STEMPRO (registered trademark) MSC SFM medium (A1050901, Thermo Fisher Scientific Inc.), the resultant cell suspension was seeded in a 2 L Erlenmeyer flask (785011, NEST Biotechnology Co., Ltd.), and shake culture was performed at a rotational speed of 70 rpm. The results are presented in Fig. 2G and Table 2. As presented in Fig. 2G and Table 2, notable cell death occurred and aggregates of dead cells or the like were observed, and therefore target cell aggregates were hardly obtained. The shear stress during the production of the cell aggregates was 0.131 Pa. In Table 2, "-" means not measured.

<Comparative Example 3>

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 400 mL of a STEMPRO (registered trademark) MSC SFM medium (A1050901, Thermo Fisher Scientific Inc.), the resultant cell suspension was seeded in a 2 L Erlenmeyer flask (785011, NEST Biotechnology Co., Ltd.), and shake culture was performed at a rotational speed of 30 rpm. The results are presented in Fig. 2H and Table 2. As presented in Fig. 2H and Table 2, the number mean diameter of the obtained aggregates was 1,000 μm or greater, and therefore it was clear that the obtained aggregates could not be expected to serve as functional cell aggregates. The shear stress during the production of the cell aggregates was 0.0028 Pa. In Table 2, "-" means not measured.

<Comparative Example 4>

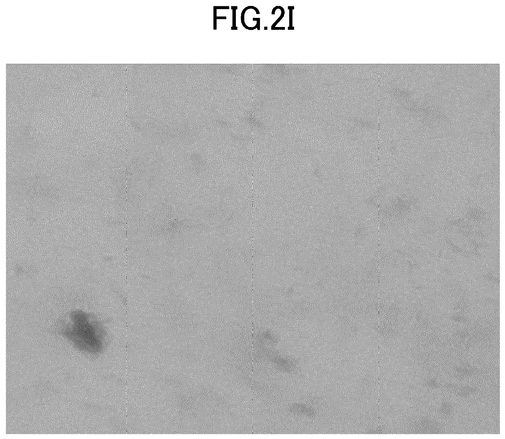

Culturing, phase-contrast microscopic observation, measurement of a number mean diameter and circularity of cell aggregates, and calculation of shear stress were performed in the same manner as in Experiment 1, except that a cell pellet having a cell number of $2 \times 10^7$ was suspended in 400 mL of a STEMPRO (registered trademark) MSC SFM medium (A1050901, Thermo Fisher Scientific Inc.), the resultant suspension was seeded in a 2 L Erlenmeyer flask (785011, NEST Biotechnology Co., Ltd.), and shake culture was performed at a rotational speed of 70 rpm. The results are presented in Fig. 2I and Table 2. As presented in Fig. 2I and Table 2, cell death occurred, and therefore a sufficient number of cell aggregates, which would allow measurement of a number mean diameter and circularity, could not be produced. The shear stress during the production of the cell aggregates was 0.46 Pa. In Table 2, "-" means not measured.

[Table 2]

| | Ex. 1 | Ex. 2 | Ex. 3 | Ex. 4 | Ex. 5 | Comp. Ex. 1 | Comp. Ex. 2 | Comp. Ex. 3 | Comp. Ex. 4 |
|---|---|---|---|---|---|---|---|---|---|
| Vessel | 250 mL flask | | | 2 L flask | | | | | |
| Medium amount (mL) | 20 | 30 | 50 | 200 | 400 | 200 | 200 | 400 | 400 |
| Shaking rotational speed (rpm) | 50 | 50 | 70 | 50 | 50 | 30 | 70 | 30 | 70 |
| Seeding cell number ($\times 10^7$) | 1 | 1 | 1.6 | 2 | 2 | 2 | 2 | 2 | 2 |
| Number mean diameter (μm) | 39 | 63 | 56 | 70 | 49 | >1000 | - | >1000 | - |
| Value of CV | 0.26 | 0.38 | 0.61 | 0.40 | 0.29 | - | - | - | - |
| Circularity | 1.57 | 1.54 | 1.64 | 1.71 | 1.79 | - | - | - | - |
| Shear stress (Pa) | 0.066 | 0.029 | 0.105 | 0.093 | 0.031 | 0.0054 | 0.131 | 0.0028 | 0.46 |

As presented in Table 2, it was found that cell aggregates having desired shapes in terms of a size, circularity, homogeneity, or the like could be obtained by setting the shear stress within the predetermined range, regardless of the size of the vessel and the amount of the medium.

As described above, it was verified that the above range of the shear stress was suitable as a parameter of the optimal culturing condition.

[Embodiment 1]

Embodiment 1 of the present disclosure is directed to a culturing-condition suggestion system that suggests culturing conditions for producing cell aggregates (spheroids). The culturing conditions in the present embodiments are culturing conditions for shake culture. The culturing conditions in the present embodiment may be culturing conditions for agitation culture. The shake culture is a method in which a vessel accommodating cells and a medium is shaken to produce spheroids. The agitation culture is a method in which contents, such as cells and a medium, accommodated in a vessel are stirred by a stirring bar to produce spheroids.

The culturing-condition suggestion system of the present embodiment aims to suggest culturing conditions in which spheroids can be efficiently produced. The culturing-condition suggestion system of the present embodiment receives input of preconditions in which one or more condition values among culturing conditions are undetermined, and outputs suggested conditions in which the one or more condition values are determined based on the preconditions.

For this, the culturing-condition suggestion system of the present embodiment determines the one or more condition values so that a predetermined indicator calculated based on the preconditions satisfies a target value that is determined in advance. Accordingly, the culturing-condition suggestion system of the present embodiment can suggest culturing conditions in which spheroids can be efficiently produced.

<Overall configuration>

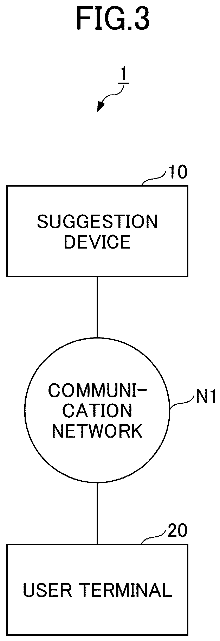

An overall configuration of the culturing-condition suggestion system of the present embodiment will be described with reference to Fig. 3. Fig. 3 is a block diagram illustrating one example of the overall configuration of the culturing-condition suggestion system of the present embodiment.

As illustrated in Fig. 3, the culturing-condition suggestion system 1 of the present embodiment includes a suggestion device 10 and a user terminal 20. The suggestion device 10 and the user terminal 20 are connected to each other via a communication network N1, such as a local area network (LAN), internet, or the like, so that data transmission can be performed between the suggestion device 10 and the user terminal 20.

The suggestion device 10 is an information processing device, such as a personal computer, a workstation, a server, or the like, that suggests culturing conditions for producing spheroids. The suggestion device 10 receives preconditions from the user terminal 20. The preconditions are culturing conditions in which one or more condition values among the culturing condition are undetermined. The suggestion device 10 determines one or more undetermined condition values based on the preconditions and generates suggested conditions including the determined condition values. The suggestion device 10 transmits the generated suggested conditions to the user terminal 20.

The user terminal 20 is an information processing terminal, such as a personal computer, a smartphone, a tablet terminal, or the like, operated by a user of the culturing-condition suggestion system 1. The user terminal 20 transmits the preconditions, in which one or more condition values are undetermined, to the suggestion device 10 in response to an operation of the user. The user terminal 20 outputs the suggested conditions received from the suggestion device 10 to the user.

The user of the culturing-condition suggestion system 1 refers to the suggested conditions output to the user terminal 20, and can produce spheroids according to the suggested conditions. The suggested conditions are suitable culturing conditions for producing spheroids, and therefore the user can efficiently produce spheroids.

Note that the overall configuration of the culturing-condition suggestion system 1 illustrated in Fig. 3 is one example, and various system configuration examples are available according to the intended use or purpose. For example, the culturing-condition suggestion system 1 may include multiple suggestion devices 10, or multiple user terminals 20, or multiple suggestion devices 10 and multiple user terminals 20. For example, the suggestion device 10 may be implemented by multiple computers, or implemented through a service of cloud computing. The classification of devices, such as the suggestion device 10 and the user terminal 20 illustrated in Fig. 3 is one example.

<Configuration of hardware>

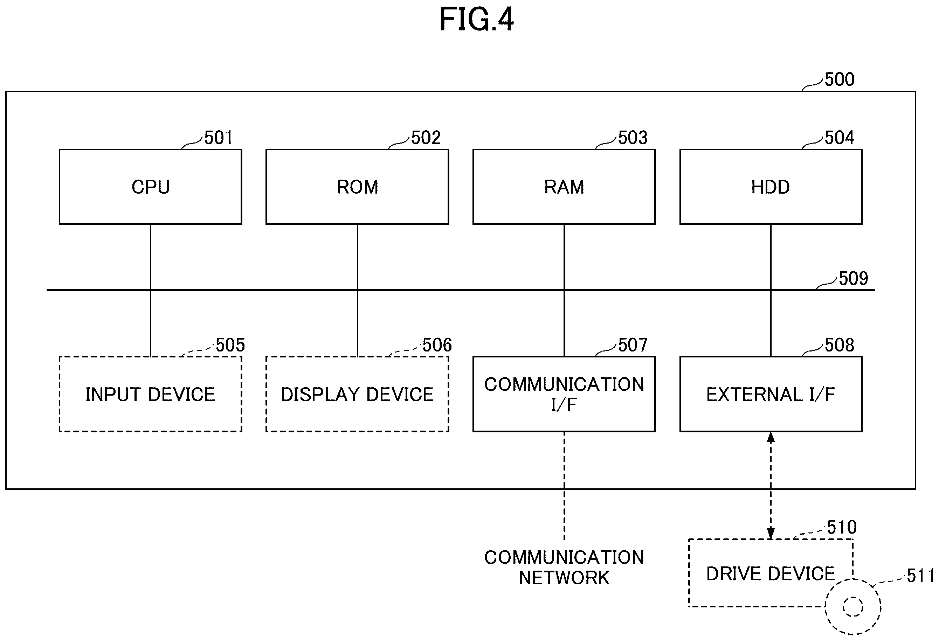

A hardware configuration of the culturing-condition suggestion system 1 of the present embodiment will be described with reference to Fig. 4.

<<Hardware configuration of computer>>

The suggestion device 10 and user terminal 20 of the present embodiment are implemented, for example, by a computer. Fig. 4 is a block diagram illustrating one example of a hardware configuration of the computer 500 of the present embodiment.

As illustrated in Fig. 4, the computer 500 includes a central processing unit (CPU) 501, a read only memory (ROM) 502, a random access memory (RAM) 503, a hard disk drive (HDD) 504, an input device 505, a display device 506, a communication interface (I/F) 507, and an external I/F 508. The CPU 501, the ROM 502, and the RAM 503 constitute what is known as a computer. The hardware components of the computer 500 are connected to each other via a bus line 509. Note that the input device 505 and the display device 506 may be configured to be used by connecting to the external I/F 508.

The CPU 501 is an arithmetic device that reads one or more programs or data from a storage device, such as the ROM 502, the HDD 504, or the like. and loads the one or more programs or data into a RAM 503 to execute processing, thereby implementing control of the entire computer 500 or functions of the computer 500.

The ROM 502 is one example of a non-volatile semiconductor memory (storage device) that can retain one or more programs or data even when a power source is turned off. The ROM 502 functions as a main storage device for storing various programs, data, and the like that are necessary for the CPU 501 to execute various programs installed in the HDD 504. Specifically, boot programs to be executed at the time of starting the computer 500, such as a basic input/output system (BIOS), an extensible firmware interface (EFI), and the like, and data for setting an operating system (OS), setting a network, and the like are stored in the ROM 502.

The RAM 503 is one example of a volatile semiconductor memory (storage device) from which programs and data are erased when the power source is turned off. For example, the RAM 503 is a dynamic random access memory (DRAM), a static random access memory (SRAM), or the like. The RAM 503 provides a work area to which various programs installed in the HDD 504 are loaded and then executed by the CPU 501.

The HDD 504 is one example of a non-volatile storage device in which programs, data, and the like are stored. The programs and data stored in the HDD 504 include OS, which is basic software for controlling the entire computer 500, applications for providing various functions on the OS, and the like. The computer 500 may utilize a storage device (e.g., a solid state drive (SSD) and the like) using a flash memory as a storage medium, instead of the HDD 504.

The input device 505 is a touch panel, operation keys, buttons, a keyboard, or a mouse used by a user to input various signals, or a microphone or the like for inputting audio data, such as voice.

The display device 506 is constituted by a display of liquid crystals, organic electro-luminescence (organic EL), or the like for displaying a screen, a speaker for outputting audio data, such as voice, and the like.

The communication I/F 507 is an interface connected to a communication network to allow the computer 500 to perform data transmission.

The external I/F 508 is an interface with one or more external devices. Examples of the external device include a drive device 510 and the like.

The drive device 510 is a device for setting a recording medium 511. The recording medium 511 includes a medium on which information is optically, electrically, or magnetically recorded, such as a CD-ROM, a flexible disk, a magneto-optical disk, or the like. In addition, the recording medium 511 may include a semiconductor memory or the like on which information is electrically recorded, such as a ROM, a flash memory, or the like. The drive device 510 allows the computer 500 to read, write, or read and write the recording medium 511 via the external I/F 508.

The various programs to be installed in the HDD 504 are installed, for example, by setting a distributed recording medium 511 in the drive device 510 connected to the external I/F 508, and reading the various programs recorded on the recording medium 511 with the drive device 510. Alternatively, the various programs to be installed in the HDD 504 may be installed by downloading the various programs through a network, which is different from the communication network, via the communication I/F 507.

<Functional configuration>

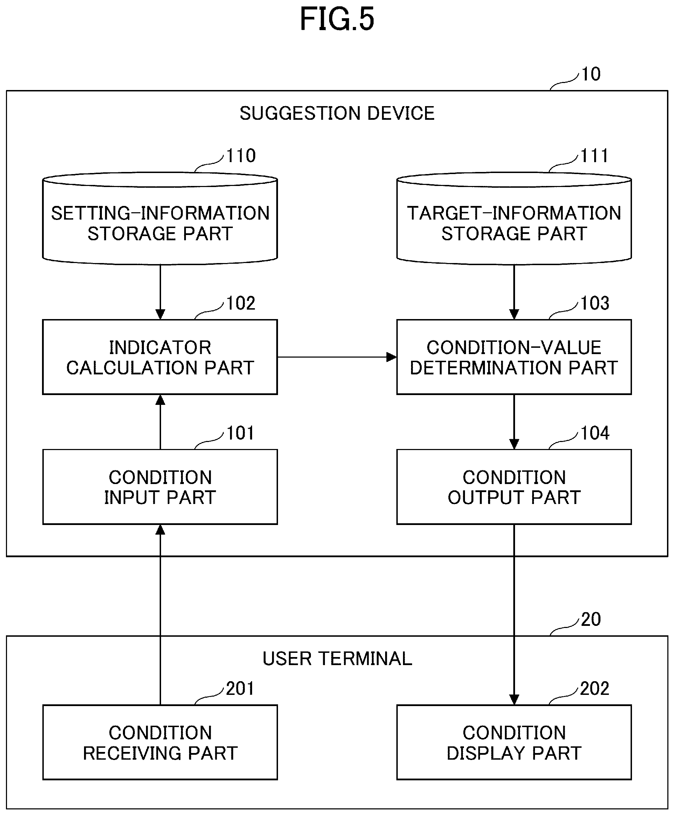

A functional configuration of the culturing-condition suggestion system 1 of the present embodiment will be described with reference to Fig. 5. Fig. 5 is a block diagram illustrating one example of the functional configuration of the culturing-condition suggestion system 1 of the present embodiment.

<<Functional configuration of suggestion device>>

As illustrated in Fig. 5, the suggestion device 10 of the present embodiment includes a condition input part 101, an indicator calculation part 102, a condition-value determination part 103, a condition output part 104, a setting-information storage part 110, and a target-information storage part 111.

The condition input part 101, the indicator calculation part 102, the condition-value determination part 103, and the condition output part 104 are implemented by allowing one or more programs, which are loaded into the RAM 503 from the HDD 504 illustrated in Fig. 4, to cause the CPU 501 to execute instructions. The setting-information storage part 110 and the target-information storage part 111 are implemented by the HDD 504 illustrated in Fig. 4.

The condition input part 101 receives input of preconditions. The condition input part 101 receives information indicating the preconditions from the user terminal 20, thereby receiving the input of the preconditions. The condition input part 101 may receive the input of the preconditions from the input device 505 or the like in response to an operation of a user.

The indicator calculation part 102 calculates a predetermined indicator based on the preconditions received by the condition input part 101. First, the indicator calculation part 102 acquires options of each condition value that is undetermined in the preconditions. The options of the condition value may be predetermined in advance, or randomly generated. Next, the indicator calculation part 102 calculates the predetermined indicator based on the culturing conditions in which the acquired condition value is set. One example of the indicator in the present embodiment is shear stress applied to cells when a vessel in which the cells and a medium are accommodated is shaken.

The condition-value determination part 103 determines the condition value, which is undetermined in the preconditions, based on the indicator calculated by the indicator calculation part 102. When the indicator is calculated for each of the options and the indicator calculated for one or more of the options satisfies a target value that is predetermined, the condition-value determination part 103 determines the one or more options of the condition value as the condition value to be included in suggested conditions.

The condition output part 104 generates suggested conditions including the condition value determined by the condition-value determination part 103. The condition output part 104 transmits information indicating the generated suggested conditions to the user terminal 20.

The setting-information storage part 110 stores setting information used for the indicator calculation part 102 to calculate the setting information. The setting information in the present embodiment is information indicating a viscosity of each of media. The viscosity of each of the media can be acquired by performing experiments in advance, or the like.

Target information is stored in advance in the target-information storage part 111, and the target information is used for the condition-value determination part 103 to determine the condition value. The target information in the present embodiment is a range of shear stress. The range of the shear stress is set, for example, at 0.0145 Pa or greater and 0.125 Pa or less. The target information stored in the target-information storage part 111 may be updated by an operation of a user at any timing.

<<Functional configuration of user terminal>>

As illustrated in Fig. 5, the user terminal 20 of the present embodiment includes a condition receiving part 201 and a condition display part 202.

The condition receiving part 201 and the condition display part 202 are implemented by allowing one or programs, which are loaded into the RAM 503 from the HDD 504 illustrated in Fig. 4, to cause the CPU 501 to execute instructions.

The condition receiving part 201 receives input of preconditions in response to an operation of a user. The condition receiving part 201 transmits information indicating the received preconditions to the suggestion device 10.

The condition display part 202 receives information indicating the suggested conditions from the suggestion device 10. The condition display part 202 displays the received suggested conditions to the display device 506.

<Process flow>

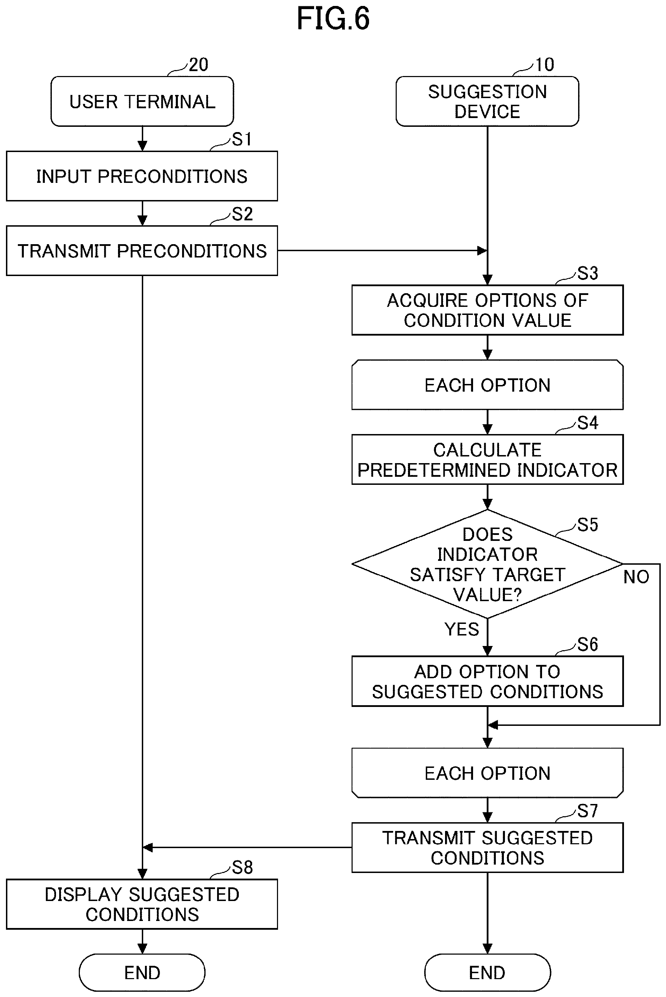

A process flow of the suggestion method performed by the culturing-condition suggestion system 1 in the present embodiment will be described with reference to Fig. 6. Fig. 6 is a flowchart illustrating one example of the process flow of the suggestion method of the present embodiment.

At step S1, the condition receiving part 201 of the user terminal 20 receives input of preconditions in response to an operation of a user. The preconditions are culturing conditions in which one or more condition values are undetermined. In the preconditions of the present embodiment, a kind of a vessel, a kind of a medium, an amount of the medium, or a rotational speed of a shaker is undetermined.

At step S2, the condition receiving part 201 of the user terminal 20 transmits the preconditions received in step S1 to the suggestion device 10. In the suggestion device 10, the condition input part 101 receives information indicating the preconditions from the user terminal 20. The condition input part 101 transmits the received preconditions to the indicator calculation part 102.

At step S3, the indicator calculation part 102 of the suggestion device 10 receives the preconditions from the condition input part 101. Next, the indicator calculation part 102 specifies one or more undetermined condition values in the received preconditions.

Subsequently, the indicator calculation part 102 acquires options of each of the one or more condition values specified. The indicator calculation part 102 may acquire the options that are predetermined based on the condition value, or may randomly determine options within a range of value that the condition value can take.

In the case where a kind of a vessel or a kind of a medium is undetermined in the preconditions, for example, information of usable vessels or information of usable media is stored in the setting-information storage part 110 or the like in advance, options in the information of the vessels or options in the information of the media can be read from the setting-information storage part 110 or the like. Moreover, in the case where an amount of a medium is undetermined in the preconditions, for example, a range from the minimum amount to the maximum amount of the medium relative to a kind of a vessel is determined in advance, and options of the amount of the medium may be determined at predetermined intervals within the determined range. Moreover, in the case where a rotational speed of a shaker is undetermined, for example, a rotational speed that can be set on a shaker to be used is stored in the setting-information storage part 110 or the like in advance, and options of the rotational speed may be read from the setting-information storage part 110 or the like.

At step S4, the indicator calculation part 102 of the suggestion device 10 generates candidate conditions in which each of the options acquired in step S3 is set as the condition value undetermined in the preconditions. Next, the indicator calculation part 102 calculates a predetermined indicator based on the generated candidate conditions. The indicator in the present embodiment is shear stress applied to cells. The indicator calculation part 102 transmits the calculated indicator to the condition-value determination part 103.

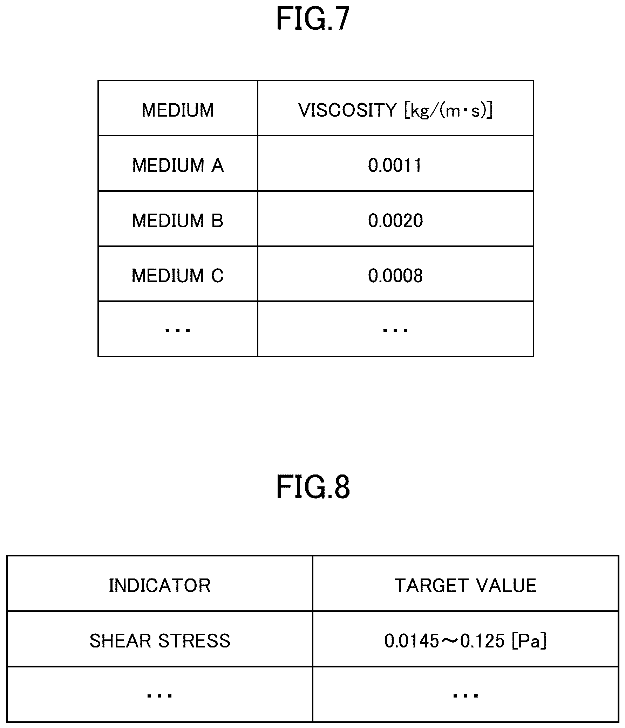

The shear stress $\tau$ [Pa] applied to cells can be calculated according to the equation (1). The viscosity $\mu$ of the medium may be read from the setting information stored in the setting-information storage part 110. Fig. 7 is a diagram illustrating one example of the setting information in the present embodiment. As illustrated in Fig. 7, the setting information of the present embodiment is information in which a kind of the medium and a viscosity [kg/(m·s)] of the medium are associated with each other.

The strain rate $\gamma$ can be calculated, for example, by computational fluid dynamic (CFD) simulation. The fluid model used in the simulation can be constructed, for example, using a volume of fluid (VOF) method.

When the fluid model is constructed, an amount of a medium and a rotational speed of a vessel used for shaking are used as parameters. A shape and size of each of the vessels are measured in advance, and are stored in the setting-information storage part 110 or the like. In the simulation, a mesh simulating a shape and size of the vessel and an amount of the medium is created, and the viscosity of the medium and the rotational speed of shaking can be set as parameters. The creation of the mesh is division of the fluid model into arbitrary small-space cells. Calculation is performed for each mesh cell, and the calculation results of all of the mesh cells are integrated to analyze the entire fluid model. A size of each mesh cell can be adjusted so that sufficient analysis accuracy is obtained, as well as considering the calculation resource and calculation time. The analysis accuracy can be improved by adjusting the mesh cells at the liquid surface boundary, the edges of the medium (contact area between the medium and the inner surface of the vessel), or both to be relatively small.

As the rotational speed of the vessel, a rotational speed set on the shaker may be used.

The description will be made referring back to Fig. 6. At step S5, the condition-value determination part 103 of the suggestion device 10 receives the indicator from the indicator calculation part 102. Next, the condition-value determination part 103 reads out the target information for the indicator from the target-information storage part 111.

Fig. 8 is a diagram illustrating one example of the target information of the present embodiment. As illustrated in Fig. 8, the target information of the present embodiment is information in which the indicator and the target value are associated with each other. The target information may be information in which the indicator and a range of the target value are associated with each other. In the present embodiment, the range of the shear stress is set at 0.0145 Pa or greater and 0.125 Pa or less.

The description will be made referring back to Fig. 6. At step S5, the condition-value determination part 103 determines whether or not the indicator calculated in step S4 satisfies the read target value. In the case where the indicator satisfies the target value (YES), the condition-value determination part 103 advances the process to step S6. In the case where the indicator does not satisfy the target value (NO), on the other hand, the condition-value determination part 103 advances the process to skip step S6.

At step S6, the condition-value determination part 103 of the suggestion device 10 transmits candidate conditions, in which the indicator satisfies the target value, to the condition output part 104. The condition output part 104 receives the candidate conditions from the condition-value determination part 103, and add the candidate conditions to suggested conditions. The suggested conditions are culturing conditions to be suggested to a user, and are culturing conditions in which the condition values are determined so that the indicator satisfies the target value.

The process from step S4 to step S6 is repeated to perform the process for each of the options acquired in step S3.

At step S7, the condition output part 104 of the suggestion device 10 transmits information indicating the suggested conditions to the user terminal 20. In the user terminal 20, the condition display part 202 receives the information indicating the suggested conditions from the suggestion device 10.

At step S8, the condition display part 202 of the user terminal 20 displays the suggested conditions on the display device 506. A user can refer to the suggested conditions displayed on the user terminal 20 and produce spheroids according to the suggested conditions. The user may review the suggested conditions displayed on the user terminal 20 and may determine new culturing conditions.

<Effects>

The suggestion device 10 of the present embodiment receives input of preconditions in which one or more condition values are undetermined, determines the one or more condition values so that an indicator calculated based on the preconditions satisfies a target value, and outputs suggested conditions including the one or more condition values that have been determined. Accordingly, the suggestion device 10 of the present embodiment can suggest culturing conditions in which the predetermined indicator satisfies the target value.

The suggestion device 10 of the present embodiment suggests, among culturing conditions for producing spheroids through shaking of a vessel in which cells and a medium are accommodated, a kind of the vessel, a kind of the medium, an amount of the medium, or a rotational speed of the shaker. Accordingly, the suggestion device 10 of the present embodiment can suggest culturing conditions in which the predetermined indicator can satisfy the target value in a method of producing spheroids through shake culture.

The suggestion device 10 of the present embodiment suggests culturing conditions in which shear stress is 0.0145 Pa or greater and 0.125 Pa or less. Accordingly, the suggestion device 10 of the present disclosure can suggest culturing conditions in which spheroids can be efficiently produced.

For example, when production of spheroids of cells in certain culturing conditions has been confirmed by an experiment or the like, production of spheroids at a large quantity may be desired using a large vessel. In this case, preconditions, in which a kind of a vessel and an amount of a medium are changed, and a rotational speed is undetermined, are input to the suggestion device 10, and a range of the rotational speed in which the shear stress falls within a range of the target value can be obtained.

Moreover, when a result of production of spheroids of cells is not desirable in certain culturing conditions, for example, it may be desired to determine a suitable kind of a medium. In this case, if preconditions in which a kind of a medium is undetermined are input to the suggestion device 10, a kind of the medium with which shear stress can satisfy the range of the target value can be determined.

[Embodiment 2]

In Embodiment 1, the configuration where the condition value, with which the indicator satisfies the target value based on the culturing conditions, is determined from options of the condition value that is undetermined in the preconditions has been described. In the second embodiment, a configuration where a condition value that is undetermined in preconditions is suggested using a learned prediction model that has learned a relationship between culturing conditions and a predetermined indicator will be described.

<Functional configuration>

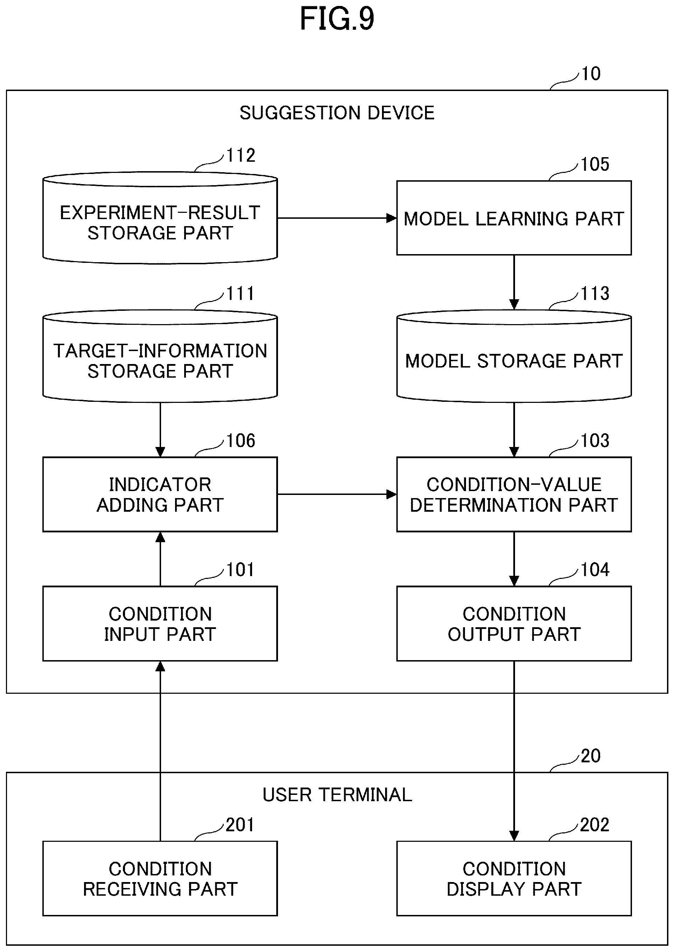

A functional configuration of the culturing-condition suggestion system 1 of the present embodiment will be described with reference to Fig. 9. Fig. 9 is a block diagram illustrating one example of the functional configuration of the culturing-condition suggestion system 1 of the present embodiment.

<<Functional configuration of suggestion device>>

As illustrated in Fig. 9, the suggestion device 10 of the present embodiment includes a condition input part 101, a condition-value determination part 103, a condition output part 104, a model learning part 105, a target-value adding part 106, a target-information storage part 111, an experiment-result storage part 112, and a model storage part 113. Specifically, the suggestion device 10 of the present embodiment is different from that of Embodiment 1 in that the suggestion device 10 of the present embodiment does not include the indicator calculation part 102 and the setting-information storage part 110, but includes the model learning part 105, the target-value adding part 106, the experiment-result storage part 112, and the model storage part 113.

In the experiment-result storage part 112, experiment results when spheroids are produced in several different culturing conditions are stored. The experiment results include the several different culturing conditions, the culturing result when spheroids are produced in each of the several different culturing conditions, and the indicator calculated based on each of the several different culturing conditions. The experiment results of the present embodiment are, for example, experiment results presented in Table 1. Note that the indicator (i.e., shear stress) included in the experiment results is calculated in advance based on the culturing conditions through simulation or the like.

In the model storage part 113, a learned prediction model which has learned a relationship between the culturing conditions and the indicator is stored. The prediction model of the present embodiment is a regression model in which, for each of condition values of the culturing conditions, the culturing conditions and the indicator are set as explanatory variables and each condition value is set as an objective variable. The prediction model may be a machine learning model, such as a neural network, random forest, or the like, which has learned to output a prediction value of a condition value for each of the condition values of the culturing conditions, when the culturing conditions and the indicator are input.

The model learning part 105 learns a prediction model based on the experiment results stored in the experiment-result storage part 112. The model learning part 105 learns the prediction model that predicts a condition value for each of the condition values of the culturing conditions. The prediction model learned by the model learning part 105 is stored in the model storage part 113.

The target-value adding part 106 adds the target value, which is read from the target-information storage part 111, to the preconditions received by the condition input part 101. In the case where the target information stored in the target-information storage part 111 indicates a range of the target value, the target-value adding part 106 may add a median value of the range of the target value, the minimum and maximum values of the range of the target value, or the target values acquired at predetermined intervals within the range of the target value.

The condition-value determination part 103 of the present embodiment predicts a condition value, which is undetermined in the preconditions, using the learned model read from the model storage part 113. As the preconditions to which the target value is added by the target-value adding part 106 are input to the prediction model, the condition-value determination part 103 determines the prediction value, which is output from the prediction model, as the condition value to be included in suggested conditions.

<Process flow>

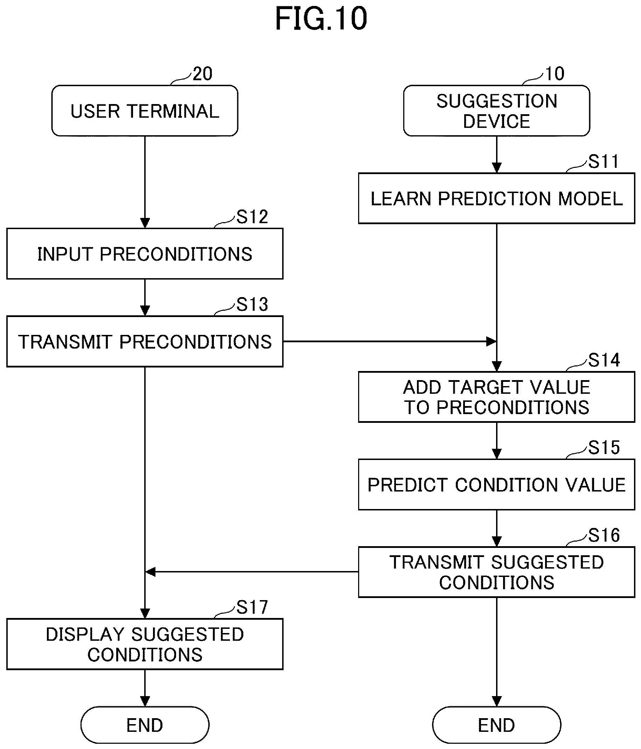

A process flow of the suggestion method performed by the culturing-condition suggestion system 1 of the present embodiment will be described with reference to Fig. 10. Fig. 10 is a flowchart illustrating one example of the process flow of the suggestion method of the present embodiment.

At step S11, the model learning part 105 of the suggestion device 10 reads out the experiment results from the experiment-result storage part 112. Next, the model learning part 105 learns a prediction model based on the read experiment results. The model learning part 105 learns a prediction model for each of condition values of the culturing conditions. Subsequently, the model learning part 105 stores the learned prediction model in the model storage part 113.

Since step S12 and step S13 are identical to step S1 and step S2 of Embodiment 1, respectively, redundant descriptions are omitted here.

At step S14, the target-value adding part 106 of the suggestion device 10 receives the preconditions from the condition input part 101. Next, the target-value adding part 106 reads out the target information from the target-information storage part 111. Subsequently, the target-value adding part 106 adds the target value to the preconditions based on the read target information. The target-value adding part 106 transmits the preconditions to which the target value is added to the condition-value determination part 103.

At step S15, the condition-value determination part 103 of the suggestion device 10 receives the preconditions, to which the target value is added, from the target-value adding part 106. Next, the condition-value determination part 103 reads out the learned prediction model from the model storage part 113. Subsequently, the condition-value determination part 103 inputs the preconditions, to which the target value is added, to the read prediction model. Thus, the condition-value determination part 103 obtains a prediction value of the condition value that is undetermined in the preconditions.

The condition-value determination part 103 generates suggested conditions by setting the prediction value, which is obtained from the prediction model, as the condition value that is undetermined in the preconditions. The condition-value determination part 103 transmits the generated suggested conditions to the condition output part 104.

<Effects>

The suggestion device 10 of the present embodiment predicts the condition value, which is undetermined in the preconditions, using the learned prediction model that has learned the relationship between the culturing conditions and the indicator. Here, the prediction of the condition value that is undetermined in the preconditions is performed by determining the condition values set in the preconditions and the target value of the indicator as explanatory variables, and the undetermined condition value as an objective variable. Accordingly, the suggestion device 10 of the present embodiment can suggest the culturing conditions in which a predetermined indicator satisfies the target value.

[Additional notes]

Each of the functions of the embodiments described above can be implemented by one or more processing circuits. In the present specification, the term "processing circuit" includes a processor that has been programed to execute each of the functions using software, such as a processor implemented by an electronic circuit, or an application specific integrated circuit (ASIC), a digital signal processor (DSP), a field programmable gate array (FPGA), or a device available in the related art, such as a circuit module, which are designed to execute each of the functions described above, and the like.

The embodiments of the present invention have been described above in detail, but the present invention is not limited to these embodiments, and various modifications or changes can be made within the scope of the gist of the present invention described in the claims.

This application claims priority to Japanese Patent Application No. 2022-199227 filed before the Japan Patent Office on December 14, 2022, the entire contents of which are incorporated herein by reference.

DESCRIPTION OF REFERENCE NUMERALS

1 culturing-condition suggestion system
10 suggestion device
101 condition input part
102 indicator calculation part
103 condition-value determination part
104 condition output part
105 model learning part
106 target-value adding part
110 setting-information storage part
111 target-information storage part
112 experiment-result storage part
113 model storage part
20 user terminal
201 condition receiving part
202 condition display part

**Claims**

1. A method of producing cell aggregates, comprising:
   culturing cells under shear stress of 0.0145 Pa or greater and 0.125 Pa or less.

2. The method of producing the cell aggregates, according to claim 1,
   wherein the cell aggregates obtained by the culturing have a number mean diameter of 400 $\mu$m or less.

3. A suggestion device, comprising:

   a condition input part configured to receive input of preconditions in which one or more condition values are undetermined among culturing conditions for producing cell aggregates;
   a condition-value determination part configured to determine the one or more condition values so that an indicator calculated based on the preconditions satisfies a target value that is determined in advance; and
   a condition output part configured to output suggested conditions including the one or more condition values that are determined.

4. The suggestion device according to claim 3,

   wherein the culturing conditions are conditions for shaking a vessel accommodating both cells and a medium to produce the cell aggregates, and
   the one or more condition values include a kind of a vessel, a kind of the medium, an amount of the medium, or a rotational speed for shaking the vessel.

5. The suggestion device according to claim 4,
   wherein the indicator is shear stress applied to the cells.

6. The suggestion device according to claim 5,
   wherein the target value is 0.0145 Pa or greater and 0.125 Pa or less.

7. The suggestion device according to any one of claims 3 to 6,
   wherein the condition-value determination part is configured to determine the one or more condition values using a learned model that has learned a relationship between the culturing conditions and the indicator.

8. The method of producing the cell aggregate, according to claim 1 or 2, further comprising:
   shaking a vessel in which both the cells and a medium are accommodated according to the suggested conditions output by the suggestion device according to any one of claims 3 to 7.

9. A suggestion method, comprising:
   causing a computer to perform:

a process of receiving input of preconditions in which one or more condition values are undetermined among culturing conditions for producing cell aggregates;

a process of determining the one or more condition values so that an indicator calculated based on the preconditions satisfies a target value that is determined in advance; and

a process of outputting suggested conditions including the one or more condition values that are determined.

10. A program for causing a computer to perform:

a process of receiving input of preconditions in which one or more condition values are undetermined among culturing conditions for producing cell aggregates;

a process of determining the one or more condition values so that an indicator calculated based on the preconditions satisfies a target value that is determined in advance; and

a process of outputting suggested conditions including the one or more condition values that are determined.

# FIG.1A

# FIG.1B

## FIG.1C

## FIG.1D

# FIG.2A

# FIG.2B

# FIG.2C

# FIG.2D

## FIG.2E

## FIG.2F

## FIG.2G

## FIG.2H

# FIG.2I

# FIG.3

# FIG.4

# FIG.5

SUGGESTION DEVICE /10

/110
SETTING-INFORMATION STORAGE PART

/111
TARGET-INFORMATION STORAGE PART

/102
INDICATOR CALCULATION PART

/103
CONDITION-VALUE DETERMINATION PART

/101
CONDITION INPUT PART

/104
CONDITION OUTPUT PART

USER TERMINAL /20

/201
CONDITION RECEIVING PART

/202
CONDITION DISPLAY PART

# FIG.6

```
        ┌──────────────┐ 20                    ┌──────────────┐ 10
        │ USER TERMINAL│                       │  SUGGESTION  │
        └──────────────┘                       │    DEVICE    │
               │                               └──────────────┘
               ▼      S1                               │
        ┌──────────────┐                               │
        │INPUT          │                              │
        │PRECONDITIONS  │                              │
        └──────────────┘                               │
               │      S2                                │
               ▼                                        │
        ┌──────────────┐                                │
        │TRANSMIT       │───────────────────────┐       │
        │PRECONDITIONS  │                        ▼       ▼
        └──────────────┘
```

- INPUT PRECONDITIONS — S1
- TRANSMIT PRECONDITIONS — S2
- ACQUIRE OPTIONS OF CONDITION VALUE — S3
- EACH OPTION
- CALCULATE PREDETERMINED INDICATOR — S4
- DOES INDICATOR SATISFY TARGET VALUE? — S5
- NO
- YES
- ADD OPTION TO SUGGESTED CONDITIONS — S6
- EACH OPTION
- TRANSMIT SUGGESTED CONDITIONS — S7
- DISPLAY SUGGESTED CONDITIONS — S8
- END
- END

# FIG.7

| MEDIUM | VISCOSITY [kg/(m·s)] |
|---|---|
| MEDIUM A | 0.0011 |
| MEDIUM B | 0.0020 |
| MEDIUM C | 0.0008 |
| ... | ... |

# FIG.8

| INDICATOR | TARGET VALUE |
|---|---|
| SHEAR STRESS | 0.0145～0.125 [Pa] |
| ... | ... |

# FIG.9

SUGGESTION DEVICE (10)

EXPERIMENT-RESULT STORAGE PART (112)

MODEL LEARNING PART (105)

TARGET-INFORMATION STORAGE PART (111)

MODEL STORAGE PART (113)

INDICATOR ADDING PART (106)

CONDITION-VALUE DETERMINATION PART (103)

CONDITION INPUT PART (101)

CONDITION OUTPUT PART (104)

USER TERMINAL (20)

CONDITION RECEIVING PART (201)

CONDITION DISPLAY PART (202)

# FIG.10

## INTERNATIONAL SEARCH REPORT

| | International application No. |
|---|---|
| | **PCT/JP2023/043739** |

### A. CLASSIFICATION OF SUBJECT MATTER

*C12N 1/00*(2006.01)i; *C12M 1/00*(2006.01)i; *C12N 5/0775*(2010.01)i; *G06N 20/10*(2019.01)i
FI: C12N1/00 B; C12M1/00 D; G06N20/10; C12N5/0775

According to International Patent Classification (IPC) or to both national classification and IPC

### B. FIELDS SEARCHED

Minimum documentation searched (classification system followed by classification symbols)

C12N1/00; C12M1/00; C12N5/0775; G06N20/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | KR 10-2020-0081331 A (KOREA RESEARCH INSTITUTE OF BIOSCIENCE AND BIOTECHNOLOGY) 07 July 2020 (2020-07-07) claims 1, 2, paragraphs [0023], [0048], [0080]-[0084], example 5.2 | 1, 2 |
| Y | | 1-10 |
| Y | JP 2017-148001 A (THE UNIVERSITY OF TOKYO) 31 August 2017 (2017-08-31) paragraph [0004] | 1-10 |
| X | JP 2021-16359 A (KANEKA CORP.) 15 February 2021 (2021-02-15) claims 1-4, 7-9, paragraphs [0006], [0054]-[0077], fig. 1-3 | 3, 7, 9, 10 |
| Y | | 3-10 |
| Y | WO 2019/181920 A1 (NIKKISO CO., LTD.) 26 September 2019 (2019-09-26) claims 1, 5, paragraph [0082] | 3-10 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 February 2024** | **13 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

International application No.

**PCT/JP2023/043739**

| Patent document cited in search report | | | Publication date (day/month/year) | Patent family member(s) | Publication date (day/month/year) |
|---|---|---|---|---|---|
| KR | 10-2020-0081331 | A | 07 July 2020 | (Family: none) | |
| JP | 2017-148001 | A | 31 August 2017 | (Family: none) | |
| JP | 2021-16359 | A | 15 February 2021 | (Family: none) | |
| WO | 2019/181920 | A1 | 26 September 2019 | US 2020/0392442 A1 claims 1, 5, paragraphs [0104]-[0107] EP 3770248 A1 | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021191305 A

- JP 2022199227 A

**Non-patent literature cited in the description**

- *Bioengineering*, 2017, vol. 4, 47

- *Frontiers in Bioengineering and Biotechnology*, 2020, vol. 8, 590332